# EUROPEAN PATENT APPLICATION

(11) **EP 1 209 235 A1**
(43) Date of publication of application: **29.05.2002**
(21) Application number: 01309792.8
(22) Date of filing: 21.11.2001
(51) Int. Cl.: C12N 15/62, C12N 11/16, C07K 14/395, C12N 9/10, C12N 15/54, C12N 15/81, C12P 19/18, C12N 1/19

(54) **Expression vector for fusion gene and method for producing immobilized enzyme**

(30) Priority: 21.11.2000 JP 2000354396; 22.06.2001 JP 2001190524
(71) Applicant: National Institute of Advanced Industrial Science and Technology, Tokyo 100-8921 (JP)
(72) Inventor: Abe, Hiroko, Tsukuba-shi, Ibaraki 305-0005 (JP); Shimma, Yoh-ichi, Tsukuba-shi, Ibaraki 305-0031 (JP); Jigami, Yoshifumi, Ushiku-shi, Ibaraki 300-1234 (JP)
(74) Representative: Maschio, Antonio

(57) **Abstract**

The present invention enables immobilization of a useful protein, for example, a glycosyltransferase, onto the surface of a yeast cell without deteriorating its enzyme activity. The present invention provides a fusion gene expression vector which is characterized by containing a fusion gene that comprises a gene encoding a useful protein bound downstream of a gene encoding a yeast cell wall protein; a transformant yeast, which is transformed with the fusion gene expression vector; and an immobilized enzyme, which is characterized in that the expressed useful protein is immobilized on a yeast cell wall.

SEQUENCE LISTING

<110> National Institute of Advanced Industrial Science

<120> Expression vector for fused gene and Method for
      producing immobilized enzyme

<130> PH-1407

<140>
<141>

<150> JP2000/354396
<151> 2000-11-21

<150> JP2001/190524
<151> 2001-06-22

<160> 14

<170> PatentIn Ver. 2.1

<210> 1
<211> 341
<212> PRT
<213> Saccharomyces cerevisiae

<400> 1
Met Gln Tyr Lys Lys Ser Leu Val Ala Ser Ala Leu Val Ala Thr Ser
  1               5                  10                  15

Leu Ala Ala Tyr Ala Pro Lys Asp Pro Trp Ser Thr Leu Thr Pro Ser
             20                  25                  30

Ala Thr Tyr Lys Gly Gly Ile Thr Asp Tyr Ser Ser Thr Phe Gly Ile
         35                  40                  45

Ala Val Glu Pro Ile Ala Thr Thr Ala Ser Ser Lys Ala Lys Arg Ala
    50                  55                  60

Ala Ala Ile Ser Gln Ile Gly Asp Gly Gln Ile Gln Ala Thr Thr Lys
65                  70                  75                  80

Thr Thr Ala Ala Ala Val Ser Gln Ile Gly Asp Gly Gln Ile Gln Ala
                85                  90                  95

Thr Thr Lys Thr Lys Ala Ala Ala Val Ser Gln Ile Gly Asp Gly Gln
            100                 105                 110

Ile Gln Ala Thr Thr Lys Thr Thr Ser Ala Lys Thr Thr Ala Ala Ala
        115                 120                 125

Val Ser Gln Ile Gly Asp Gly Gln Ile Gln Ala Thr Thr Lys Thr Lys
    130                 135                 140

Ala Ala Ala Val Ser Gln Ile Gly Asp Gly Gln Ile Gln Ala Thr Thr
145                 150                 155                 160

Lys Thr Thr Ala Ala Ala Val Ser Gln Ile Gly Asp Gly Gln Ile Gln
                165                 170                 175

Ala Thr Thr Lys Thr Thr Ala Ala Ala Val Ser Gln Ile Gly Asp Gly
            180                 185                 190

Gln Ile Gln Ala Thr Thr Asn Thr Thr Val Ala Pro Val Ser Gln Ile
        195                 200                 205

Thr Asp Gly Gln Ile Gln Ala Thr Thr Leu Thr Ser Ala Thr Ile Ile
    210                 215                 220

Pro Ser Pro Ala Pro Ala Pro Ile Thr Asn Gly Thr Asp Pro Val Thr
225                 230                 235                 240

Ala Glu Thr Cys Lys Ser Ser Gly Thr Leu Glu Met Asn Leu Lys Gly
                245                 250                 255

Gly Ile Leu Thr Asp Gly Lys Gly Arg Ile Gly Ser Ile Val Ala Asn
            260                 265                 270

Arg Gln Phe Gln Phe Asp Gly Pro Pro Pro Gln Ala Gly Ala Ile Tyr
        275                 280                 285

Ala Ala Gly Trp Ser Ile Thr Pro Glu Gly Asn Leu Ala Ile Gly Asp
    290                 295                 300

Gln Asp Thr Phe Tyr Gln Cys Leu Ser Gly Asn Phe Tyr Asn Leu Tyr
305                 310                 315                 320

Asp Glu His Ile Gly Thr Gln Cys Asn Ala Val His Leu Gln Ala Ile
                325                 330                 335

Asp Leu Leu Asn Cys
            340

<210> 2
<211> 413
<212> PRT
<213> Saccharomyces cerevisiae

<400> 2
Met Gln Tyr Lys Lys Thr Leu Val Ala Ser Ala Leu Ala Ala Thr Thr
1               5                   10                  15

Leu Ala Ala Tyr Ala Pro Ser Glu Pro Trp Ser Thr Leu Thr Pro Thr
            20                  25                  30

Ala Thr Tyr Ser Gly Gly Val Thr Asp Tyr Ala Ser Thr Phe Gly Ile

35                  40                  45

Ala Val Gln Pro Ile Ser Thr Thr Ser Ser Ala Ser Ser Ala Ala Thr
    50                  55                  60

Thr Ala Ser Ser Lys Ala Lys Arg Ala Ala Ser Gln Ile Gly Asp Gly
65                  70                  75                  80

Gln Val Gln Ala Ala Thr Thr Thr Ala Ser Val Ser Thr Lys Ser Thr
                85                  90                  95

Ala Ala Ala Val Ser Gln Ile Gly Asp Gly Gln Ile Gln Ala Thr Thr
            100                 105                 110

Lys Thr Thr Ala Ala Ala Val Ser Gln Ile Gly Asp Gly Gln Ile Gln
        115                 120                 125

Ala Thr Thr Lys Thr Thr Ser Ala Lys Thr Thr Ala Ala Ala Val Ser
    130                 135                 140

Gln Ile Ser Asp Gly Gln Ile Gln Ala Thr Thr Thr Thr Leu Ala Pro
145                 150                 155                 160

Lys Ser Thr Ala Ala Ala Val Ser Gln Ile Gly Asp Gly Gln Val Gln
                165                 170                 175

Ala Thr Thr Thr Thr Leu Ala Pro Lys Ser Thr Ala Ala Ala Val Ser
            180                 185                 190

Gln Ile Gly Asp Gly Gln Val Gln Ala Thr Thr Lys Thr Thr Ala Ala
        195                 200                 205

Ala Val Ser Gln Ile Gly Asp Gly Gln Val Gln Ala Thr Thr Lys Thr
    210                 215                 220

Thr Ala Ala Ala Val Ser Gln Ile Gly Asp Gly Gln Val Gln Ala Thr

225                 230                 235                 240

Thr Lys Thr Thr Ala Ala Ala Val Ser Gln Ile Gly Asp Gly Gln Val
                245                 250                 255

Gln Ala Thr Thr Lys Thr Thr Ala Ala Ala Val Ser Gln Ile Thr Asp
            260                 265                 270

Gly Gln Val Gln Ala Thr Thr Lys Thr Thr Gln Ala Ala Ser Gln Val
        275                 280                 285

Ser Asp Gly Gln Val Gln Ala Thr Thr Ala Thr Ser Ala Ser Ala Ala
    290                 295                 300

Ala Thr Ser Thr Asp Pro Val Asp Ala Val Ser Cys Lys Thr Ser Gly
305                 310                 315                 320

Thr Leu Glu Met Asn Leu Lys Gly Gly Ile Leu Thr Asp Gly Lys Gly
                325                 330                 335

Arg Ile Gly Ser Ile Val Ala Asn Arg Gln Phe Gln Phe Asp Gly Pro
            340                 345                 350

Pro Pro Gln Ala Gly Ala Ile Tyr Ala Ala Gly Trp Ser Ile Thr Pro
        355                 360                 365

Asp Gly Asn Leu Ala Ile Gly Asp Asn Asp Val Phe Tyr Gln Cys Leu
    370                 375                 380

Ser Gly Thr Phe Tyr Asn Leu Tyr Asp Glu His Ile Gly Ser Gln Cys
385                 390                 395                 400

Thr Pro Val His Leu Glu Ala Ile Asp Leu Ile Asp Cys
                405                 410

<210> 3
<211> 44
<212> DNA
<213> Artificial Sequence

<220>
<223> Description of Artificial Sequence:Artificial
      Sequence

<400> 3
ggggggagct catgcaatac aaaaaatcat tagttgcctc cgcc                    44


<210> 4
<211> 31
<212> DNA
<213> Artificial Sequence

<220>
<223> Description of Artificial Sequence:Artificial
      Sequence

<400> 4
cccccgcggc cgcacagtgc aaatcgatag c                                 31


<210> 5
<211> 35
<212> DNA
<213> Artificial Sequence

<220>
<223> Description of Artificial Sequence:Artificial
      Sequence

<400> 5

ggggggcggc cgcataccca tacgatgttc ctgac 35

<210> 6
<211> 47
<212> DNA
<213> Artificial Sequence

<220>
<223> Description of Artificial Sequence:Artificial
      Sequence

<400> 6
ggggcccggg ctaggatgat ggtttcaaaa gattttgaat atgatcc 47

<210> 7
<211> 35
<212> DNA
<213> Artificial Sequence

<220>
<223> Description of Artificial Sequence:Artificial
      Sequence

<400> 7
cccgtcgaca atcctatctg cgtgtgtctc aagac 35

<210> 8
<211> 35
<212> DNA
<213> Artificial Sequence

<220>
<223> Description of Artificial Sequence:Artificial

Sequence

<400> 8
cccctcgagt caggtgaacc aagccgctat gccgc                                35

<210> 9
<211> 39
<212> DNA
<213> Artificial Sequence

<220>
<223> Description of Artificial Sequence:Artificial
      Sequence

<400> 9
ggggggtcga cagcaatata ttccgagttc catctccgc                            39

<210> 10
<211> 42
<212> DNA
<213> Artificial Sequence

<220>
<223> Description of Artificial Sequence:Artificial
      Sequence

<400> 10
   gggggctcga gctactcacg gaattttttc cagttttttg gc                          42

<210> 11
<211> 38
<212> DNA
<213> Artificial Sequence

<220>
<223> Description of Artificial Sequence:Artificial
      Sequence

<400> 11
ggggggagct catgcaatac aaaaagactt tggttgcc                              38


<210> 12
<211> 68
<212> DNA
<213> Artificial Sequence

<220>
<223> Description of Artificial Sequence:Artificial
      Sequence

<400> 12
cccccgcggc cgccttgtca tcgtcatcct tgtagtcaca gtctatcaaa tcgatagctt 60
ccaagtgg                                                              68


<210> 13
<211> 49
<212> DNA
<213> Artificial Sequence

<220>
<223> Description of Artificial Sequence:Artificial
      Sequence

<400> 13
ggggggcggc cgcaaatgat gcgcttatac gatcaagcaa tgtaaacag                 49

<210> 14
<211> 38
<212> DNA
<213> Artificial Sequence

<220>
<223> Description of Artificial Sequence:Artificial
      Sequence

<400> 14
gggggcccgg gctagctttg ttcgtgtcta gaattttc                              38

## Description

### FIELD OF THE INVENTION

The present invention relates to a fusion gene expression vector which enables expression of a Pir (protein internal repeat) protein having ability to be localized and immobilized on a yeast cell wall in the form of a fusion protein comprising the Pir protein bound to the N-terminus of a desired, useful protein; yeast transformed with the expression vector; an immobilized enzyme comprising a useful protein immobilized on the yeast cell wall; and a method for producing a sugar chain or sugars using a glycosyltransferase immobilized on the above yeast cell wall, wherein the useful protein is the glycosyltransferase.

### BACKGROUND OF THE INVENTION

An enzyme protein used for producing substances with a bioreactor or the like is generally used as an immobilized enzyme which is immobilized to an insoluble carrier, for the sake of the convenience of procedures and from an economical standpoint. A general method for producing an immobilized enzyme involves purifying enzyme proteins and immobilizing the proteins to resin beads or the like. However, mass purification of enzyme proteins may not only involve complex procedures, but also cost a great deal, and cause inactivation of enzyme activity during a purification process. Further, a process to immobilize to a carrier, e.g., resin beads, may also inactivate enzyme activity. There is another example of a method which immobilizes an intact cell producing an enzyme protein, and uses it as an immobilized enzyme. However in this case, the efficacy of enzyme reaction cannot be said to be optimum since the enzyme protein is located within a cell. Hence, it is desired to localize an enzyme protein on the surface of a cell that is a carrier.

One of the binding patterns of plasma membrane proteins to a membrane involves addition of the glycolipid, GPI (glycosylphosphatidylinositol), to the C-terminus of a protein which is anchored to a membrane by the lipid portion of GPI (GPI anchor) embedded within the membrane (Conzelmann, EMBO J. 7, 2233-2240, (1988)). It has been reported that immobilization of a cell wall protein (GPI-anchored protein) having the GPI anchor onto a cell wall comprises cleavage and removal of the C-terminal portion of a protein in the endoplasmic reticulum during the protein biosynthesis, addition of a GPI anchor to the protein, modification of a GPI core sugar chain in the Golgi apparatus, transportation to a cell membrane, transfer of the GPI glycan to a cell wall glucan, and covalent attachment to the cell wall (Lu, J. Cell, Biol. 128, 333-340, (1995)). Thus, a GPI anchor is a useful means to immobilize a protein to the surface layer of a cell. For example, an established technique employs α-agglutinin that is anchored by GPI to a cell wall to immobilize a foreign protein to a cell wall (Schreuder et al., Trends Biotechnol., 14, 115-120, (1996)). However, a defect of GPI anchors is that a GPI anchor functions for localization and immobilization to a cell wall only when a GPI attachment signal and the C-terminus of a protein are fused, and does not function when the signal and the C-terminus are not fused (Lipke, Mol. Cell Biol. 9, 3155-3165, (1989)). As a method to avoid such drawback, a modified method of immobilization to a cell surface layer with a GPI anchor has been reported which employs association of two subunits composing an a-agglutinin, AGA1p (Roy, Mol Cell Biol., 11, 4196-4206, (1991)) and AGA2p (Cappellaro, EMBO J., 10, 4081-4088, (1991)) on the surface layer of a cell (Boder and Wittrup, Nature Biotechnol. 15, 553-557, 1997) . In this method, a fusion protein (to which a target protein has been linked to the C-terminal side of AGA2p) is bound to AGA1p (which has been immobilized on a cell wall by a GPI anchor) via S-S bonding on the cell wall so as to immobilize the target protein to the cell surface layer. However, this method can be applied only to a cell expressing AGA1p.

On the other hand, human hormones, most physiologically active substances and the receptors of these physiologically active substances consist of proteins and sugar chains. The sugar chain portion plays an important role in physiological activity, and a protein body alone cannot exhibit its original function (Akira Kobata, Protein, Nucleic Acid and Enzyme, 36, 775-788 (1991)). A glycosyltransferase is an essential enzyme in the formation of a sugar chain structure. It is thought that there are several hundred types of human glycosyltransferase. There must therefore be an enormous number of different types of glycosyltransferases when those of microorganisms and plants are included. A glycosyltransferase possesses an extremely high substrate specificity so that it adds a certain sugar to a receptor sugar chain with a certain structure by a certain binding pattern, thereby synthesizing a sugar chain with a certain structure (Qwens, Biochem. Biophys. Res. Commun., 109, 1075-1082, (1982); Betteridge, Eur. J. Biochem., 132, 29-35, (1983)). That is, the structure of a sugar chain is infinitely diversified such that a variety of sugars are associated by various binding patterns including branching. The diversity of the structure is determined by the combination of the substrate specificities of various glycosyltransferases. Accordingly, production and utilization of complex carbohydrates requires efficient expression and preparation of a glycosyltransferase that synthesizes a necessary sugar chain structure, because a substance functioning in vivo cannot be produced without precise control of the sugar chain structure.

Most glycosyltransferases are type II membrane proteins, and are localized over the membrane of the Golgi apparatus with a topology in which an active region on the C-terminal side is oriented to the lumen of the Golgi apparatus (Paulson, J. Biol. Chem., 264, 17615-17618, 1989). Therefore, when the C-terminal side of a glycosyltransferase is genetically altered, the enzyme activity will often be deteriorated. It is very difficult to immobilize a glycosyltransferase onto a cell wall while maintaining its enzyme activity, using the above-described GPI anchor that cannot function unless it is bound to the C-terminal side. Meanwhile, the N-terminal side of a glycosyltransferase contains a transmembrane region, by which the enzymes are localized to ER or Golgi membrane. Soluble glycosyltransferase forms are also present in biological fluid. They arise from proteolytic cleavage in the stem region. That is, addition of a novel anchor protein (for immobilization to cell surface layer) to a glycosyltransferase lacking a transmembrane region would be effective in controlling localization of glycosyltransferases.

A Pir (protein with internal repeat) protein is a cell wall protein covalently bound to a yeast cell wall, and Pir 1 to 4 genes, which are homologous to each other, compose a family (TOH-E, YEAST, 9, 481-494, (1993) ; Mrsa, YEAST, 13, 1145-1154, (1997)). However, a Pir protein has no GPI-anchor attachment signal. Further, since Pir proteins are not eluted with a detergent, but break away from a cell wall under alkali conditions, they may be localized and bound to a cell wall by a binding mechanism different from those of GPI-anchored proteins and other non-covalently proteins, and their mechanism remains unknown (Mrsa, YEAST, 15, 813-820, (1999)).

There has been an attempt to use Pir as an anchor protein, in which a protein A gene is inserted between genes encoding Pir 4 proteins to be localized on a cell wall (Moukadiri et al., J. Bacteriology, 181, 4741-4740, (1999)). However the binding pattern to a cell wall is so varied among Pir proteins that Pir proteins cannot be generalized. For example, Pir4 can be freed from a cell wall by β-mercaptoethanol treatment, while Pir1 and Pir2 cannot be freed by the same treatment but can be eluted from a cell wall only under alkali conditions. Therefore, Pir 4 is thought to differ from other Pir1, Pir2 and Pir3, in the binding mechanism to a cell wall (Moukadiri et al., J. Bacteriology, 181, 4741-4740, (1999)). Further, a protein fused to Pir4 is a protein A, which does not require a very precise structure compared to a protein, for example, an enzyme, and is not fused to the N-terminal side of a target protein. Hence, when a protein to be expressed is, for example, an enzyme protein, has activity closely related to its structure, use of a gene encoding a Pir4 protein as an anchor protein does not always cause the resulting, expressed protein to retain enzyme activity.

### SUMMARY OF THE INVENTION

The purpose of the present invention is to localize and immobilize a protein represented by a glycosyltransferase, which is deteriorated by genetic manipulation of the C-terminus, on the surface layer of a yeast cell wall while maintaining its activity, and provide the protein as an immobilized enzyme.

As a result of thorough studies to solve the above problems, the inventors have completed the present invention by finding that a fusion protein comprising a Pir protein bound to the N-terminus of a useful protein can be expressed on the surface layer of a yeast cell wall while maintaining the activity of the useful protein by transformation of yeast with a fusion gene expression vector containing the fusion gene that comprises a gene encoding the useful protein downstream of a gene encoding the Pir protein of a yeast cell wall.

That is, the present invention is as described in the following (1) to (16), by which the above problems are solved.
(1) A fusion gene expression vector which contains a fusion gene comprising a gene encoding a useful protein downstream of a gene encoding the following yeast cell wall protein (a) or (b) :
   (a) a protein having an amino acid sequence represented by SEQ ID NO: 1;
   (b) a protein having an amino acid sequence derived from the amino acid sequence represented by SEQ NO: 1 by deletion, replacement, or addition of one or more amino acids, and having ability to be localized and immobilized on a yeast cell wall.
(2) The fusion gene expression vector of (1) wherein the useful protein is a glycosyltransferase protein.
(3) A transformant yeast which is transformed by the fusion gene expression vector of (1) or (2).
(4) A method for producing an immobilized enzyme, which comprises culturing the transformant yeast of (3), expressing a fusion gene on the surface layer of a yeast cell wall, and obtaining yeast that contains a useful protein immobilized on the cell wall.
(5) An immobilized enzyme, which is obtained by the method of (4).
(6) The immobilized enzyme of (5) wherein the enzyme to be immobilized is a glycosyltransferase.
(7) A method for producing a sugar chain or sugars which method employs the immobilized enzyme of (6).
(8) A fusion gene expression vector which contains a fusion gene comprising a gene encoding a useful protein bound downstream of a gene encoding the following yeast cell wall protein (a) or (b) :
   (a) a protein having an amino acid sequence represented by SEQ ID NO: 2;
   (b) a protein having an amino acid sequence derived from the amino acid sequence represented by SEQ NO: 2 by deletion, replacement, or addition of one or more amino acids, and having ability to be localized and immobilized on a yeast cell wall.
(9) The fusion gene expression vector of (8) wherein the useful protein is a glycosyltransferase protein.
(10) A transformant yeast, which is transformed with the fusion gene expression vector of (8) or (9).
(11) A method for producing an immobilized enzyme which method comprises culturing the transformant yeast of (10), expressing a fusion gene on the surface layer of a yeast cell wall, and obtaining yeast that contains a useful protein immobilized on the cell wall.
(12) An immobilized enzyme, which is obtained by the method of (11).
(13) The immobilized enzyme of (12) wherein the enzyme to be immobilized is a glycosyltransferase.
(14) A method for producing a sugar chain or sugars which method employs the immobilized enzyme of (13).
(15) A transformant yeast which is transformed by allowing the yeast to contain at least two or more types of the fusion gene expression vector of (1) and (8).
(16) A method for producing an immobilized enzyme which method comprises culturing the transformant yeast of (15), expressing simultaneously fusion genes on the surface layer of a yeast cell wall, and obtaining yeast that contains at least two or more types of useful proteins immobilized on the cell wall.
(17) An immobilized enzyme, which is obtained by the method of (16).
(18) The immobilized enzyme of (17) wherein the enzymes to be immobilized are two or more types of glycosyltransferases.
(19) A method for producing a sugar chain or sugars which method sequentially converts sugar chains or sugars using the immobilized enzymes of (18).

### BRIEF DESCRIPTION OF THE DRAWINGS

Figure 1 shows a structure of YEp352GAP-II (PIR1-HA-gma12)(pAB4).
Figure 2 shows results of detecting expression of a fusion protein for a transformant strain W303-YEp352GAP-II(PIR1-HA-gma12) and a control strain W303-YEp352GAP-II by the indirect immunofluorescence technique.
Figure 3 shows results of detecting galactosyltransferase activity for a transformant strain W303-YEp352GAP-II(PIR1-HA-gma12) and a control strain W303-YEp352GAP-II. The tip of an arrow in the figure indicates a peak of galactosyl mannobiose.
Figure 4 shows the structure of YEp352GAP-II(PIR1-HA-FUT6)(pAB9).
Figure 5 shows results of detecting expression of a fusion protein for a transformant strain W303-YEp352GAP-II (PIR1-HA-FUT6) and a control strain W303-YEp352GAP-II.
Figure 6 shows results of detecting fucosyltransferase activity for a transformant strain W303-YEp352GAP-II (PIR1-HA-FUT6) and a control strain W303-YEp352GAP-II. The tip of an arrow in the figure indicates a peak of Lacto-N-fucopentaose.
Figure 7 shows the structure of YEp352GAP-II(PIR1-HA-KRE2).
Figure 8 shows the structure of YEp352GAP-II(PIR2-FLAG-MNN1).
Figure 9 indicates simultaneous expression of YEp352GAP-II (PIR1-HA-KRE2) and YEp351GAP-II(PIR2-FLAG-MNN1) in transformant yeast.
Figure 10 indicates that a yeast strain [ W303-YEp352GAP-II(PIR1-HA-KRE2), YEp351GAP-II(PIR2-FLAG-MNN1)] which has been transformed with Pir1-HA-Kre2 and Pir2-FLAG-Mnn1 at the same time performed sequential transfer reaction of mannose resulting from the two expression vectors above.

### DETAILED DESCRIPTION OF THE INVENTION

A fusion gene expression vector of the present invention contains a fusion gene which comprises a gene of a desired enzyme protein downstream of a gene encoding a Pir protein that is present on a yeast cell wall having an amino acid sequence of SEQ ID NO: 1 or 2.

PIR genes used in the present invention include a gene (PIR1) encoding a Pir1 protein represented by SEQ ID NO: 1, and a gene (PIR2) encoding a Pir2 protein represented by SEQ ID NO: 2, and a gene encoding a protein having an amino acid sequence derived from the amino acid sequence above by deletion, replacement or addition of one or more amino acids and having ability to be localized or immobilized to a yeast cell wall.

Examples of an amino acid sequence derived from the amino acid sequence represented by SEQ ID NO: 1 or 2 by deletion, replacement or addition of one or more amino acids include: an amino acid sequence which is derived from the amino acid sequence of SEQ ID NO: 1 or 2 by deletion of one to 10 amino acids, preferably 1 to 5 amino acids, and more preferably 1 to 2 amino acids; an amino acid sequence which is derived from the amino acid sequence of SEQ ID NO: 1 or 2 by replacement of one to 10 amino acids, preferably 1 to 5 amino acids, and more preferably 1 to 2 amino acids by other amino acids; and an amino acid sequence which is derived from the amino acid sequence of SEQ ID NO: 1 or 2 by addition of one to 10 amino acids, preferably 1 to 5 amino acids, and more preferably 1 to 2 amino acids.

Examples of such an amino acid sequence derived from the amino acid sequence represented by SEQ ID NO: 1 or 2 by deletion, replacement or addition of one or more amino acids have at least 80% or more, preferably 90% or more, more preferably 95% or more, and particularly preferably 97% or more homology with the amino acid sequence of SEQ ID NO: 2 when homology is calculated with BLAST.

Such an amino acid sequence derived from the amino acid sequence represented by SEQ ID NO: 1 or 2 by deletion, replacement or addition of one or more amino acids is substantially identical to the amino acid sequence of SEQ ID NO: 1 or 2.

More specifically, these genes can be obtained by PCR using a genome obtained from budding yeast (*Saccharomyces cerevisiae*) strain W303-1A (ura3, leu2, his3, trp1, ade2) [Kainuma et al., Glycobiology, Vol. 9, 133-141 (1999)) as a template. A primer used herein contains a restriction enzyme cleavage portion, which is useful for easy cleavage of a portion encoding a Pir protein and simple insertion of a epitope tag sequence and the like.

Any desired, useful protein gene may be downstream of the above PIR gene. For example, a gene of a protein such as a glycosyltransferase whose activity may be deteriorated by gene manipulation of the C-terminus, is preferred. Examples of such a protein include a galactosyltransferase, fucosyltransferase, glucosyltransferase, mannosyltransferase, galactosamyltransferase, sialyltransferase and N-acetylglucosaminyltransferase.

An expression cassette is constructed by inserting a promoter to upstream and a terminator to downstream of a fusion gene comprising a desired protein gene downstream of a PIR gene. Then, the expression cassette is introduced into an vector. Alternatively, when a promoter and a terminator have already been present in an expression vector into which the fusion gene is introduced, only the fusion gene is introduced between the promoter and the terminator without constructing any expression cassette.

For a promoter in an expression cassette, any promoter which is generally used for a yeast expression system and allows expression of a fusion gene introduced in a transformed yeast cell can be used. Examples of such a promoter include, but are not specifically limited to, include PGK, GAP, TPI, GAL1, GAL10, ADH2, PH05 and CUP1. Particularly, a GAP promoter is preferred.

For a terminator, any terminator which is generally used for a yeast expression system and allows termination of transcription when it is located downstream of a fusion gene introduced can be used. Examples of such a terminator include ADH1, TDH1, TFF and TRP5.

Examples of an expression vector to which an expression cassette is introduced are not specifically limited as long as they are generally used in a yeast expression system, and allow expression of a fusion gene on the surface layer of the cell wall of transformant yeast that has been transformed with the expression vector. For example, a yeast episome expression vector can be preferably used.

A yeast episome plasmid vector contains a 2µ plasmid sequence, which is an original sequence of yeast and the vector is rendered capable of replication within a host yeast cell using the autonomously replicating sequence of the 2µ plasmid sequence. Examples of a yeast episome expression vector used in the present invention are not specifically limited, as long as they contain at least an ARS sequence of the yeast 2µ plasmid sequence and they can replicate outside the chromosome within a host yeast cell. Such a yeast episome expression vector may be YEp51, pYES2, YEp351, YEp352 or the like.

The above yeast episome expression vector is preferably a shuttle vector, which can proliferate within *E.coli* cells to perform subcloning in recombinant *E.coli.* A more preferred expression vector contains a selective marker gene, such as an ampicillin-resistantgene. In addition, the expression vector contains a marker gene with which a yeast clone can be selected based on auxotrophy and drug resistance when recombinant yeast is produced. Examples of a marker gene include HIS3, TRP1, LEU2, URA3, ADE2, CAN1, SUC2, LYS2, and CUP1 (Yasuji Oshima (writer-editor), Experimental Protocols in Biochemistry 39, Experimental Protocols in Yeast Molecular Genetics, 119-144 (1996)). These are merely examples, and selection should be made depending on a genotype of a yeast strain to be used as a host for gene transfer.

The above series of techniques involved in construction of a fusion gene expression plasmid may be appropriately performed by persons skilled in the art by referring to descriptions given in examples described later or by standard technology.

In this invention, examples of host yeast to be transformed with the above fusion gene expression vector include, but are not limited to, yeast belonging to the genus *Saccharomyces* and the genus *candida*. Examples of yeast belonging to the genus *Saccharomyces* include *Saccharomyces cerevisiae* strains KK4, Y334, Inv-Sc1 and W303.

To transform yeast with a fusion gene expression vector, for example, known methods such as a lithium acetate method, electroporation, and the like can be used (Becker and Guarente, Methods Enzymol., 194, 182-187 (1991) ).

In the present invention, yeast may be transformed simultaneously using multiple expression vectors in which genes encoding different useful proteins are bound to the same type of PIR genes. Yeast may also be transformed simultaneously using multiple expression vectors in which genes encoding different useful proteins are bound respectively to the different types of PIR genes (e.g. PIR1 gene and PIR2 gene). In these cases, for example, by using as genes encoding useful proteins multiple genes (different, but related to each other) encoding glycosyltransferase proteins, immobilized enzymes of the transformed yeast can perform multiple reactions sequentially. Hence, these cases have an advantage that greatly diverse sugar chains or sugars can be produced.

Here, the term "immobilized enzyme" means an enzyme immobilized to a yeast cell wall.

An appropriate selective marker is used for screening for transformant yeast. In a preferred example, a gene involved in the metabolism on a chromosomal DNA of a host cell is used. That is, when transforming a host cell having the above gene on chromosomal DNA which has been disabled function by appropriate techniques, e.g., mutant with an expression vector that contains a corresponding normal gene, a preferred selection marker is one which can be used to screen by proliferating the transformant cell that contains a normal metabolism gene. More specifically, a widely used selective marker gene, for example, URA3, and LEU2 as described above is integrated to an expression vector. For a chromosome incorporation type (YIp type), these genes are also used as markers for screening.

Culturing of the transformed transformant yeast enables expression of a fusion protein comprising a Pir protein bound to the N-terminus of a desired protein onto the surface layer of the cell wall. The desired protein is immobilized via Pir on the surface layer of the cell wall of the transformant yeast, so that the transformant yeast can be used directly as an immobilized enzyme.

Culturing of transformant yeast can be performed by standard techniques for culturing yeast.
A medium used herein contains a yeast assimilable carbon source, a nitrogen source, inorganic salts and the like to enable efficient culturing of transformants. For example, a synthetic medium that can be used herein (containing a carbon source, a nitrogen source, inorganic salts, amino acids, vitamins and the like) contains various medium components (supplied from Difco) added thereto, except amino acid (required for replication and maintenance of plasmids, but can be supplied with a marker) which is removed from the medium (Sherman, Methods Enszymol., 194, 3-57 (1991)).

pH for a medium is preferably adjusted to 6 to 8. Adjustment of pH is performed using inorganic or organic acid, alkali solution, urea, calcium carbonate, ammonia or the like.

Culturing is performed at 28 to 32°C, preferably at 30°C, for 15 to 48 hours with aeration and agitation.

### EXAMPLE

Now, the present invention will be further described by the following examples, but is not limited thereto.

### Example 1 (Cloning of budding yeast PIR1 gene)

PIR1 gene was isolated by PCR using the genome obtained from a budding yeast (*Saccharomyces cerevisiae*) strain W303-1A (ura3, leu2, his3, trp1, ade2) [Kainuma et al., Glycobiology, Vol. 9, 133 (1999)] as a template.

A primer was designed based on a base sequence which has been registered on a database (DB name: GenBank; Accession NO: D13740). At this time, a primer previously containing an SacI site at the N-terminal portion and an NotI site at the C-terminal portion was so designed that a portion encoding a protein can be easily cleaved out with a restriction enzyme and a epitope tag sequence and the like can be simply inserted. Base sequences for each primer are as follows.

### Forward primer:

5'-GGGGGGAGCTCATGCAATACAAAAAATCATTAGTTGCCTCCGCC-3'
(SEQ ID NO: 3)

### Reverse primer:

5'-CCCCCGCGGCCGCACAGTGCAAATCGATAGC
(SEQ ID NO: 4)

The underlined portion in the base sequence of the forward primer indicates the SacI site; and that of the reverse primer indicates the NotI site. Thus, the designed primers were synthesized by standard techniques.

Composition of the PCR solution is as shown in Table 1.

**Table 1**

| Composition of PCR solution | |
|---|---|
| 10 x ExpandHF buffer (including 15mM MgCl₂) | 10µl |
| dNTP Mixture (2.5mM each) | 8µl |
| Forward primer (20pmpl / µl) | 2µl |
| Reverse primer (20pmpl / µl) | 2µl |
| Genome DNA | 3µl |
| ExpandTM High Fidelity PCR System enzyme mix | 0.75µl |
| Water | 74.25µl |
| Total | 100µl |

The first PCR reaction consisted of 10 cycles. The temperature conditions for each cycle consisted of denaturation of template DNA at 94°C for 2 min, 94°C for 15 sec (denaturation), 50°C for 30 sec (annealing), and 72°C for 1 min (elongation). The next reaction consisted of 15 cycles, the temperature conditions for each cycle consisting of 94°C for 15 sec (denaturation), 50°C for 30 sec (annealing), and 72°C for 1 min (elongation) and each cycle having a prolongation of 5 sec. Finally, elongation reaction at 72°C was performed for 7 min. The amplified DNA fragment with a length of approximately 1kbp obtained by the PCR was cleaved with NotI-SacI, and then inserted to an NotI-SacI site of pBluescript II SK(-) (Stratagen), thereby constructing pBSII (PIR1) (pAB2).

### Example 2 (Construction of fusion protein composed of fission yeast Gma12 and HA Tag)

A gma12 gene (DB name: GenBank; Accession No: Z30917) (Chappell, Mol. Biol. Cell, 5, 519-528 (1994)) of fission yeast (*Schizosaccharomyces pombe*) was also cloned by PCR. Composition of the PCR solution is shown in Table 2.

**Table 2**

| Composition of PCR solution | |
|---|---|
| 10 x ExpandHF buffer (including 15mM MgCl₂) | 10µl |
| dNTP Mixture (2.5mM each) | 8µl |
| Forward primer (20pmpl / µl) | 2µl |
| Reverse primer (20pmpl / µl) | 2µl |
| Plasmid DNA | 1µl |
| ExpandTM High Fidelity PCR System enzyme mix | 0.75µl |
| Water | 76.25µl |
| Total | 100µl |

The plasmid (pYD1-HA-gma12) that has been constructed from gma12 gene cloned into YEpU-GAP-gma12 (YOKO-O et al., FEBS, Vol.257, 1998) by Takayama who belonged to the inventors' laboratory was used as a template. A primer was so designed to enable amplification of HA-gma12 fusion gene that the amplified product previously contains an NotI site on the 5' side and an SmaI site on the 3' side. Primers having the following base sequences were used.

### Forward primer:

5'-GGGGGGCGGCCGCATACCCATACGATGTTCCTGAC
(SEQ ID NO: 5)

### Reverse primer:

5'-GGGGCCCGGGCTAGGATGATGGTTTCAAAAGATTTTGAATATGATCC
(SEQ ID NO: 6)

The underlined portion in the base sequence of the forward primer indicates the NotI site; that of the reverse primer indicates the SmaI site. PCR was performed under temperature conditions the same as those employed for amplification of PIR1 by PCR.

Subsequently, the HA-gma12 fusion gene was inserted to an NotI-SmaI site of pBluescript II SK(-) (Stratagene), thereby constructing pBSII (HA-gma12) (pAB1).

### Example 3 (Preparation of PIR1-HA-gma12 fusion gene, fusion gene expression vector, and transformant yeast containing the plasmid)

A PIR1 gene inserted in pBSII(PIR1) was cleaved with SacI-NotI, and then inserted into an SacI-NotI site of pBSII (HA-gma12), thereby constructing pBSII (PIR1-HA-gma12) (pAB3).

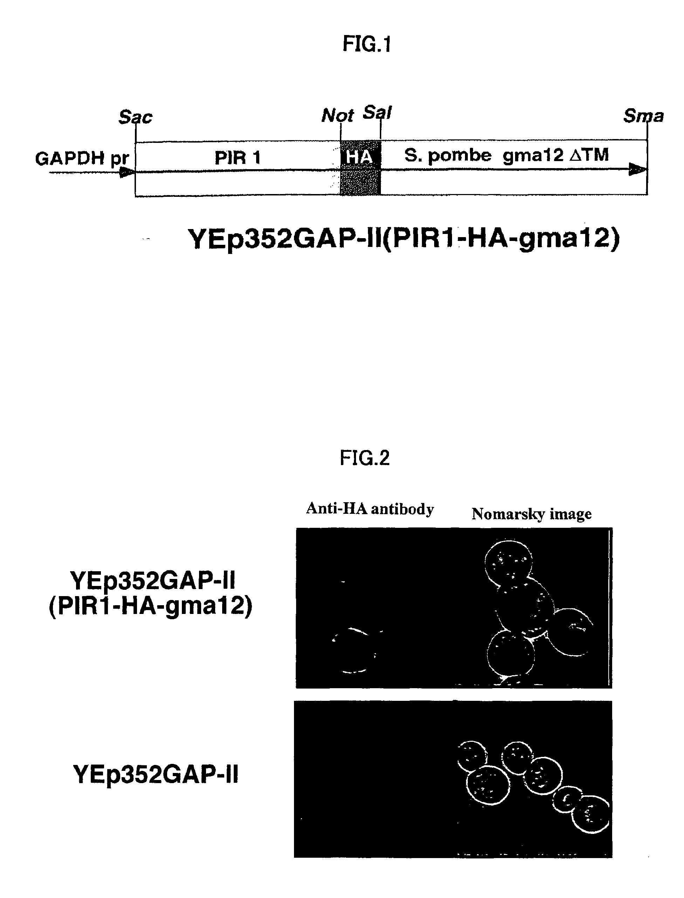

A PIR1-HA-gma12 portion of the pBSII (PIR-HA-gma12) was cleaved with SacI-SmaI. Then the product was inserted into an SacI-SmaI site of a expression vector YEp352GAP-II (Nakayama) in which the multi-cloning site of a yeast expression vector YEp352GAP (Roy et al., J. Biol. Chem., Vol.237, 2538 (1998)) has been replaced by a portion from EcoRI to SalI of the multi-cloning site of pUC18, thereby constructing YEp352GAP-II (PIR1-HA-gma12) (pAB4) (Fig. 1).

The expression vector YEp352GAP-II (PIR1-HA-gma12) was transformed to a yeast strain W303-1A (ura3, leu2, his3, trp1, ade2) [Kainumaetal., Glycobiology, 9, 133-141 (1999)], thereby obtaining a strain W303- YEp352GAP-II(PIR1-HA-gma12).

In addition, the strain W303- YEp352GAP-II(PIR1-HA-gma12) was deposited under the Accession No. FERM BP-7794 at the International Patent Organism Depository, National Institute of Advanced Industrial Science and Technology on November 16, 2000.

### Example 4 (Expression of Pir1-HA-Gma12 fusion protein within yeast cells)

The transformant obtained in Example 3 was examined by the indirect immunofluorescence technique to ascertain whether a fusion gene was expressed within a cell and a fusion protein was presented on the surface layer of the yeast cell.

First, the above transformant strain W303-YEp352GAP-II(PIR1-HA-gmal2) and a control strain W303-YEp352GAP-II, which had been transformed from a strain W303-1A using YEp352GAP-II, were cultured in 5ml of a SD (-uracil) liquid medium up to OD600=5 (for approximately 30 hours). Then, 1ml of the culture solution was collected, and then the cells were washed with PBS [8mg / ml NaCl, 0.2mg / ml KCl, 1.44mg / ml Na₂HPO₄, 0.24mg/ml KH₂PO₄ (pH7.4)]. The cells were collected, suspended in 250µl of a PBS solution [8mg / ml NaCl, 0.2mg / ml KCl, 1.44mg / ml Na₂HPO₄, 0.24mg / ml KH2PO4 (pH7.4) , 1mg / ml BSA] containing 1µg of HA antibody [Anti-HA High Affinity (Roche)], and then incubated on ice for 30 min. The cells were collected and washed once with a PBS solution. Subsequently, the cells were suspended in 250µl of a PBS solution [8mg / ml NaCl, 0.2mg / ml KCl, 1.44mg / ml Na₂HPO₄, 0.24mg / ml KH₂PO₄ (pH7.4), 1mg/ml BSA] containing 1µg of a fluorescein secondary antibody [Alexa FluorTM 546 goat anti-rat IgG (H+L) conjugate (Molecular Probe)], and then incubated on ice for 30 min while shielding from light. During their respective incubation for 30 min, the cells and the antibody solution were occasionally mixed by a turning-over method for thorough mixing. The cells were collected, washed twice with PBS, suspended in 40µl of PBS, and then observed with a fluorescence microscope (Fig. 2).

As a result, expression of Pir1-HA-Gma12 fusion protein on the cell surface layer was confirmed for the strain W303-YEp352GAP-II(PIR1-HA-gma12).

### Example 5 (Measurement of galactosyltransferase activity)

Galactosyltransferase activity was measured by referring to Yoko-O et al's method (Yoko-O, Eur. J. Biochem., 257, 630-637 (1998)). As an enzyme source, the yeast intact cell itself [W303-YEp352GAP-II(PIR1-HA-gma12)] prepared in Example 3 was used. As an acceptor substrate, PA-mannobiose was used; as a donor substrate, UDP-galactose was used. A reaction solution of 50µl [100mM HEPES (pH7.2) , 1mM MnCl₂, 5mM UDP-galactose, 300pmol PA-mannobiose] was prepared to contain 11µl of a cell suspension, followed by incubation at 37°C for 5 hours. The cell suspension used herein was prepared by collecting 1ml of a culture solution with OD600=6, washing twice with Wash Buffer [10mM Tris-HCl (pH8) , 1mM PMSF], and suspending in 11µl of Wash Buffer [10mM Tris-HCl (pH8) , 1mM PMSF]. Then, 30µl of ice-cooled water was added to the reaction solution. The precipitated cells were removed by centrifugation at 3,000rpm for 3 min, the supernatant with a molecular weight of 10,000 or more was removed with an Ultra Free (0.22µm), and then mannobiose and galactosylmannobiose were measured with HPLC. An Amide-80 column (TSK gel Amide-80, TOSOH, 0.46cm in diameter x 25cm in length) was used for HPLC. A mixture A containing 200mM acetic acid-triethylamine buffer (pH 7.0) and acetonitrile (10 : 90), and a mixture B containing 200mM acetic acid-triethylamine buffer (pH 7.0) and acetonitrile (60 : 40) were prepared. The column had been previously equilibrated by running the solvent A through the column at a flow rate of 1.0ml/min. Immediately after injection of samples, the proportion of the solvent B was raised linearly for 60 min up to 100%, so that PA-oligosaccharide was eluted.

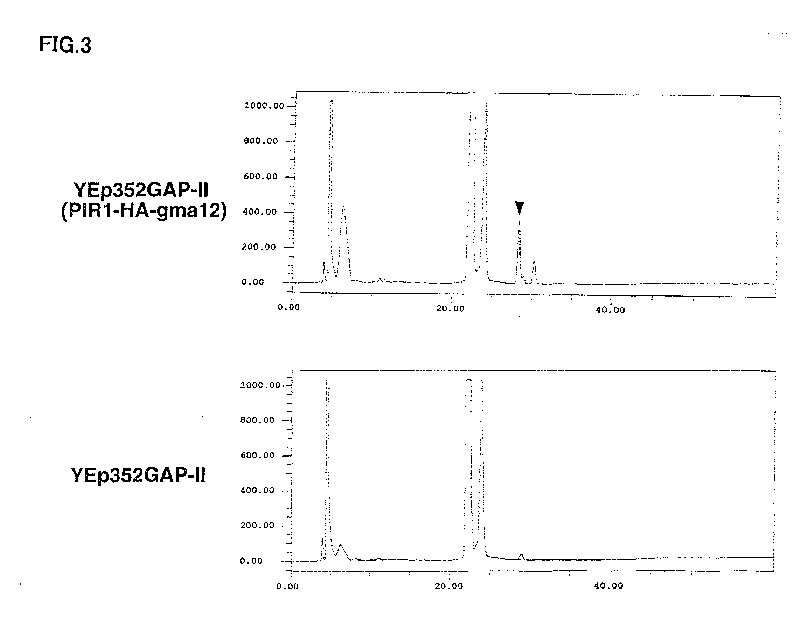

As a result, a peak of the enzyme product was detected, that is, galactosyltransferase activity could be confirmed, only for the strain W303-YEp352GAP-II (PIR1-HA-gma12) that can express a fusion protein of Pir1-HA-gma12 (Fig. 3). No galactosyltransferase activity was detected for the strain W303-YEp352GAP-II that expresses no fusion gene.

### Example6 (Preparation of PIR1-HA-FUT6 fusion gene, fusion gene expression vector, and yeast transformant containing the plasmid)

A plasmid pBS(SK-)/FT6H1.3 (provided by Dr. Narumatsu of Soka University) containing the amino acid coding region of a FUT6 gene, which is a human α-1,3-FucT (DB name: GenBank; AccessionNo:L01698) (Weston, J. Biol. Chem., 267, 24575-24585 (1992)) , was used as a template. A primer previously containing an SalI site on the N-terminal side and an XhoI site on the C-terminal side was designed to enable amplification except for a transmembrane region located on the N-terminal side of an FUT6 protein. Primers having the following base sequences were used.

### Forward primer:

5'-CCCGTCGACAATCCTATCTGCGTGTGTCTCAAGAC-3'
(SEQ ID NO: 7)

### Reverse primer:

5'-CCCCTCGAGTCAGGTGAACCAAGCCGCTATGCCGC-3'
(SEQ ID NO: 8)

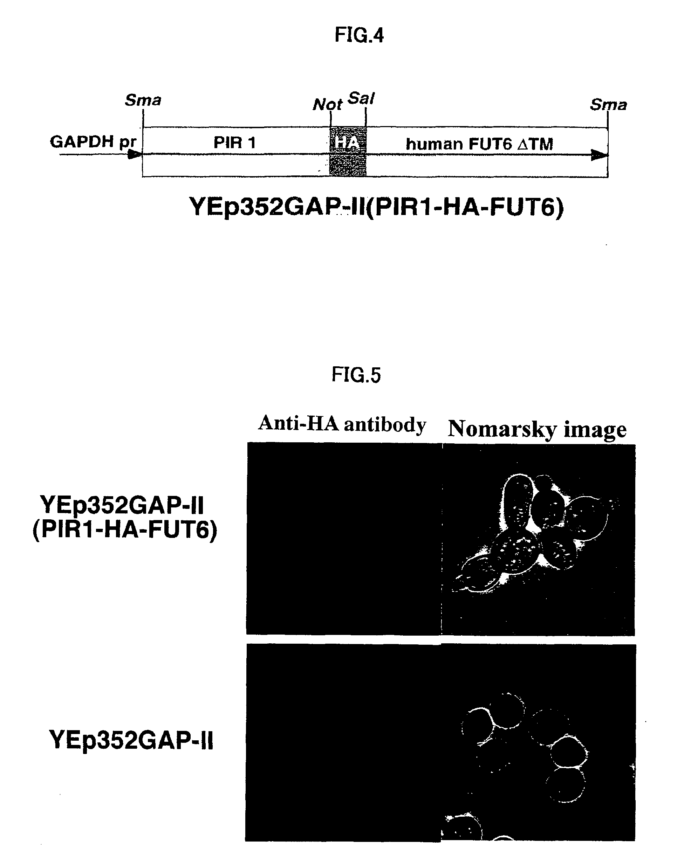

The underlined portion in the base sequence of the forward primer indicates the SalI site; that of the reverse primer indicates the XhoI site. The composition of a reaction solution and the reaction conditions employed for PCR followed Table 2 of Example 1 and the reaction conditions for Example 1, respectively. The amplified fragment of approximately 1kb was insertedin-frame to the SalI-XhoI site of pBSII(PIR1-HA-gma12), thereby constructing pBSII(PIR1-HA-FUT6)(pAB7). The PIR1-HA-FUT6 portion was cleaved with SacI-XhoI from the pBSII(PIR1-HA-FUT6), blunt-ended with Blunting high (TOYOBO), and then inserted into an SmaI site of an expression vector YEp 352GAP-II (provided by Nakayama of the inventors' laboratory), thereby constructing YEp352GAP-II(PIR1-HA-FUT6)(pAB9) (Fig. 4).

The expression vector YEp352GAP-II(PIR1-HA-FUT6) was transformed to a yeast strain W303-1A (ura3, leu2, his3, trp1, ade2) [Kainuma et al., Glycobiology, Vol. 9, 133-141 (1999)], thereby obtaining a strain W303- YEp352GAP-II(PIR1-HA-FUT6).

In addition, the strain W303- YEp352GAP-II(PIR1-HA-FUT6) was deposited under the Accession No. FERM BP-7797 at the International Patent Organism Depository, National Institute of Advanced Industrial Science and Technology on November 16, 2000.

### Example 7 (Expression of Pir1-HA-FUT6 fusion protein within yeast cell)

The transformant obtained in Example 6 was examined by the indirect immunofluorescent technique to ascertain whether a fusion gene was expressed within a cell and a fusion protein was presented on the surface layer of the yeast cell.

First, the above transformant strain W303-YEp352GAP-II(PIR1-HA-FUT6) and a control strain W303-YEpGAP-II, which had been transformed from a strain W303-1A using YEp352GAP-II, were cultured in 5ml of an SD (-uracil) liquid medium to OD600=5 (for approximately 30 hours). Then, 1ml of the culture solution was collected, and then the cells were washed with PBS [8mg / ml NaCl, 0.2mg / ml KCl, 1.44mg / ml Na₂HPO₄, 0.24mg / ml KH₂PO₄ (pH7.4) ]. The cells were collected, suspended in 250µl of a PBS solution [8mg / ml NaCl, 0.2mg / ml KCl, 1.44mg / ml Na₂HPO₄, 0.24mg / ml KH₂PO₄ (pH7.4) , 1mg / ml BSA] containing 1µg of HA antibody [Anti-HA High Affinity (Roche)], and then incubated on ice for 30 min. The cells of each strain were collected respectively and washed once with a PBS solution. Subsequently, the cells were suspended in 250µl of a PBS solution [8mg / ml NaCl, 0.2mg / ml KCl, 1.44mg / ml Na₂HPO₄, 0.24mg / ml KH₂PO₄ (pH7.4) , 1mg / ml BSA) containing 1µg of a labeled secondary antibody [Alexa FluorTM 546 goat anti-rat IgG (H+L) conjugate (Molecular Probe)], and then incubated on ice for 30 min while shielding from light. During their respective incubation for 30 min, the cells and the antibody solution were mixed occasionally by a turning-over method for thorough mixing. The cells were collected, washed twice with PBS, suspended in 40µl of PBS, and then observed with a fluorescence microscope.

As a result, expression of Pir1-HA-FUT6 fusion protein on the cell surface layer was confirmed for the strain W303-YEp352GAP-II(PIR1-HA-FUT6) (Fig. 5).

### Example 8 (Measurement of fucosyltransferase activity)

Fucosyltransferase activity was measured by referring to GLYCOBIOLOGY Experimental Protocol (ed. Taniguchi et al., 156-159 (1996)). As an enzyme source, a solution was prepared by disrupting yeast cells [W303―YEp352GAP-II(PIR1-HA-FUT6), prepared in Example 6] with glass beads in Wash Buffer [10mM Tris-HCl (pH8), 1mM PMSF]. PA-Lacto-N-neotetraose was used as an acceptor substrate; GDP-fucose was used as a donor substrate. 5.5µl of the cell disruption solution was added to 4.5µl of a reaction solution [50mM Cacodylate buffer (pH 6.8), 5mM ATP, 25mM MnCl₂, 0.075mM GDP-fucose, 0.075mM PA-Lacto-N-neotetraose], followed by incubation at 37°C for 5 hours. A cell suspension used herein was a solution containing disrupted cells which had been prepared by collecting 0.25ml of the culture solution with OD600=6, washing twice with Wash Buffer [10mM Tris-HCl (pH 8), 1mM PMSF] and then crushing with glass beads. Next, to stop reaction, incubation was performed at 98°C for 3 min, and then 40µl of ice-cooled water was added to the reaction solution. The precipitated cells were removed by centrifugation at 3,000rpm for 3 min, and then the supernatant with a molecular weight of 10,000 or more was removed with an Ultra Free (0.22µm). Subsequently, Lacto-N-neotetraose and Lacto-N-fucopentaose were measured with HPLC. An Amide-80 column (TSK gel Amide-80, TOSOH, 0.46cm in diameter x 25cm in length) was used for HPLC. A mixture A containing 200mM acetic acid-triethylamine buffer (pH 7.0) and acetonitrile (10 : 90), and a mixture B containing 200mM acetic acid -triethylaminebuffer (pH7.0) andacetonitrile (60 : 40) were prepared. The column had been previously equilibrated by running the solvent A through the column at a flow rate of 1.0ml/min. Immediately after injection of samples, the proportion of the solvent B was raised linearly for 60 min to 100%, so that PA-oligosaccharide was eluted.

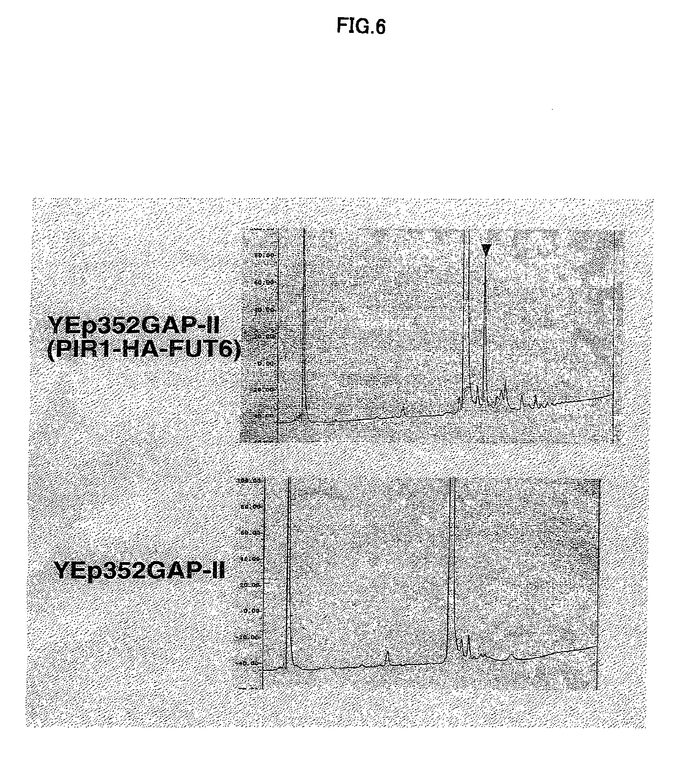

As a result, a peak of the enzyme product was detected, that is, fucosyltransferase activity could be confirmed, only for the strain W303-YEp352GAP-II (PIR1-HA-FUT6) that can express a fusion protein of Pir1-HA-FUT6 (Fig. 6). No fucosyltransferase activity was detected for the strain W303-YEp352GAP-II that expresses no fusion gene.

### Example 9 (Preparation of PIR1-HA-KRE2 fusion gene and fusion gene expression vector)

A KRE2 gene was isolated by PCR using the genome obtained from a budding yeast strain W303-1A (ura3, leu2, his3, trp1, ade2) [Kainuma et al., Glycobiology, Vol. 9, 133-141 (1999)] as a template.

Primers were designed based on a base sequence registered on a database (DB name: GenBank; Accession No: X62647). At this time, the primers previously containing an SalI site on the N-terminal side and an XhoI site on the C-terminal side were designed to enable amplification without the transmembrane region located on the N-terminal side. Primers having the following base sequences were used.

### Forward primer :

5'-GGGGGGTCGACAGCAATATATTCCGAGTTCCATCTCCGC-3'
(SEQ ID NO: 9)

### Reverse primer:

5'-GGGGGCTCGAGCTACTCACGGAATTTTTCCAGTTTTTGGC-3'
(SEQ ID NO: 10)

Here, the underlined portion in the base sequence of the forward primer indicates the SalI site; that of the reverse primer indicates the XhoI site. Thus the designed primers were synthesized by standard techniques.

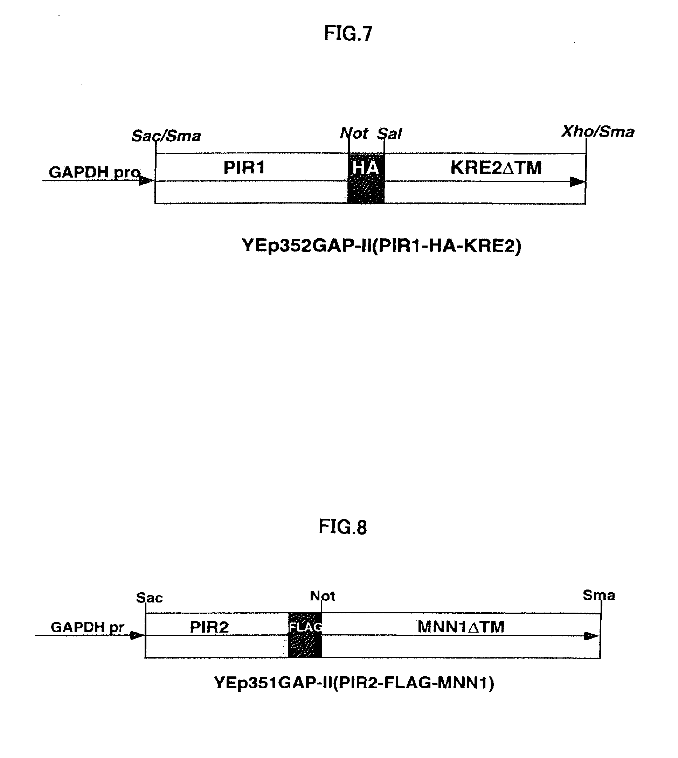

The composition of a reaction solution and the reaction conditions employed for PCR followed Table 1 of Example 1 and the reaction conditions of Example 1, respectively. The amplified fragment of approximately 1kb was inserted in-frame to the SalI-XhoI site of pBSII(PIR1-HA-gma12), thereby constructing pBSII(PIR1-HA-KRE2)(pAB27). The PIR1-HA-KRE2 portion was cleaved with SacI-XhoI from the pBSII(PIR1-HA-KRE2), blunt-ended with Blunting high (TOYOBO), and then inserted into an SmaI site of an expression vector YEp352GAP-II (provided by Nakayama of the inventors laboratory), thereby constructing YEp352GAP-II(PIR1-HA-KRE2) (pAB30) (Fig. 7).

### Example 10 (Construction of fusion protein composed of budding yeast Pir2 and FLAG)

A PIR2 gene was isolated by PCR using the genome obtained from a budding yeast strain W303-1A (ura3, leu2, his3, trp1, ade2) [Kainuma et al., Glycobiology, Vol. 9, 133-141 (1999)] as a template.

Primers were designed based on a base sequence registered on a database (DB name: GenBank; Accession No: D13741). At this time, the primers previously containing an SacI site at the N-terminal portion and the sequence of FLAG epitope tag sequence containing an NotI site at the C-terminal portion were designed to enable easy cleavage of a portion encoding a protein with a restriction enzyme and to enable a epitope tag sequence to be added to the C-terminal portion of a Pir2 protein. The base sequences of each primer were as follows.

### Forward primer:

5'-GGGGGGAGCTCATGCAATACAAAAAGACTTTGGTTGCC-3'
(SEQ ID NO: 11)

### Reverse primer:

5'-CCCCCGCGGCCGCCTTGTCATCGTCATCCTTGTAGTCACAGTCTATCAAATCG
ATAGCTTCCAAGTGG-3'
(SEQ ID NO: 12)

The underlined portion in the base sequence of the forward primer indicates the SacI site; that of the reverse primer indicates the NotI site. The box portion indicates a sequence of a FLAG epitope tag. Thus the designed primers were synthesized by standard techniques.

The composition of a reaction solution and the reaction conditions employed for PCR followed Table 1 of Example 1 and the reaction conditions of Example 1, respectively. The amplified fragment of approximately 1kb was cleaved with SacI-NotI, and then inserted to the SacI-NotI site of pBluescript II SK(-) (Stratagene), thereby constructing pBSII(PIR2-FLAG)(pAB22).

### Example 11 (Preparation of PIR2-FLAG-MNN1 fusion gene and fusion gene expression vector)

An MNN1 gene was isolated by PCR using the genome obtained from a budding yeast strain W303-1A (ura3, leu2, his3, trp1, ade2) [Kainuma et al., Glycobiology, Vol. 9, 133-141 (1999)] as a template.

Primers were designed based on a base sequence registered on a database (DB name: GenBank; Accession No: L23753). At this time, the primers previously containing an NotI site on the N-terminal side and an SmaI site on the C-terminal side were designed to enable amplification without the transmembrane region located on the N-terminal side. Primers having the following base sequences were used.

### Forward primer:

5'-GGGGGGCGGCCGCAAATGATGCGCTTATACGATCAAGCAATGTAAACAG-3'
(SEQ ID NO: 13)

### Reverse primer:

5'-GGGGGCCCGGGCTAGCTTTGTTCGTGTCTAGAATTTTC-3'
(SEQ ID NO: 14)

The underlined portion in the base sequence of the forward primer indicates the NotI site; that of the reverse primer indicates the SmaI site. Thus, the designed primers were synthesized by standard techniques.

The composition of a reaction solution and the reaction conditions employed for PCR followed Table 1 of Example 1 and the reaction conditions of Example 1, except that the time for elongation reaction was changed from 1 to 2 min. The amplified fragment of approximately 2kb was inserted in-frame to the NotI-SmaI site of pBSII(PIR2-FLAG) (pAB22), thereby constructing pBSII (PIR2-FLAG-MNN1) (pAB28). The PIR2-FLAG-MNN1 portion was cleaved with SacI-SmaI from the pBSII(PIR2-FLAG-MNN1), and then inserted into an SacI-SmaI site of an expression vector YEp352GAP-II (provided by Nakayama of the inventors laboratory), thereby constructing YEp352GAP-II(PIR2-FLAG-MNN1)(pAB29). Further, to construct a plasmid having a leucine marker, a Bg1I fragment containing a promoter region, PIR2-FLAG-MNN1 and a terminator region was cleaved from Yep352GAP-II (PIR2-FLAG-MNN1), and then inserted into a Bg1I site of self amplification vector YEp351 [Hill et al., YEAST, 2, 163-167 (1986)], thereby constructing YEp351GAP-II(PIR2-FLAG-MNN1) (pAB31)(Fig. 8).

### Example 12 (Construction of yeast transformant containing expression vector YEp352GAP-II(PIR1-HA-KRE2) and YEp351GAP-II(PIR2-FLAG-MNN1), and expression of both fusion proteins in yeast cells)

The expression vectors YEp352GAP-II (PIR1-HA-KRE2) and YEp351GAP-II(PIR2-FLAG-MNN1) were transformed simultaneously to a yeast strain W303-1A (ura3, leu2, his3, trp1, ade2) [Kainuma et al., Glycobiology, 9, 133-141(1999)) , thereby obtaining a strain W303-YEp352GAP-II(PIR1-HA-KRE2), YEp351GAP-II(PIR2-FLAG-MNN1). The strain W303-YEp352GAP-II(PIR1-HA-KRE2),
YEp351GAP-II(PIR2-FLAG-MNN1) was deposited under the Accession No. FERM BP-7789 at the International Patent Organism Depository, National Institute of Advanced Industrial Science and Technology on June 20, 2001.

The obtained transformant was subjected to Western blotting to examine if fusion genes were co-expressed in a cell and fusion proteins were co-localized on the yeast cell surface layer. PIR protein was covalently attached to a cell wall by an alkali-sensitive linkage, suggesting that a fusion protein with PIR would be freed by a mild alkali treatment of the cell wall fraction.

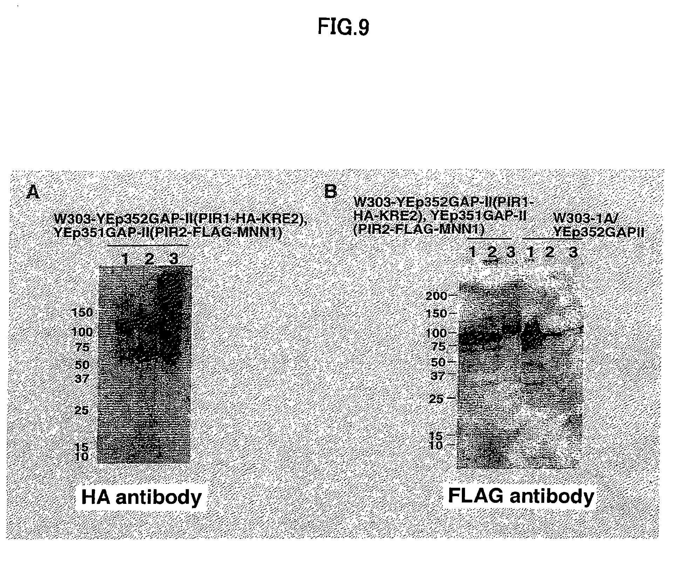

First, the above transformant strain W303-YEp352GAP-II(PIR1-HA-KRE2),
YEp351GAP-II(PIR2-FLAG-MNN1) and a control strain W303-YEp352GAP-II, which had been transformed from a strain W303-1A using YEp352GAP-II, were cultured in 5ml each of an SD (-uracil, -leucine) and an SD(-uracil) liquid media to OD600=5 (for approximately 48 hours). Then, the cells were collected and washed with Wash Buffer [10mM Tris-HCl (pH8.0), 1mM PMSF]. Glass beads were added to the cell suspension and Voltex was applied at 4°C for 15 min, so that the cells were disrupted. The solution containing the disrupted cells was separated into supernatant (Lane 1 of Fig. 9A and B) and pellet. The pellet was washed three times with Wash Buffer [10mM Tris-HCl (pH8.0), 1mM PMSF], suspended in 100µl of Laemmli Buffer [4% SDS, 20% glycerol, 0.12M Tris-HCl (pH6.6), 8M urea, 2%β-ME], and then boiled at 100°C for 10 min. This sample was centrifuged and further separated into supernatant (Lane 2 of Fig. 9A and B) and pellet fraction. The pellet fraction was washed three times with Na-acetate Buffer (pH 5.5) [0.1M Na-acetate], and then incubated with 100µl of a mild alkali solution [30mM NaOH] at 4°C for 15 hours. The suspension with the mild alkali solution was centrifuged, so that supernatant was collected (Lane 3 of Fig. 9A and B). The collected samples were subjected to SDS-PAGE, and then Western blotting. At this time, primary antibodies used herein were HA antibody [MONOCLONAL ANTIBODY,HA.11 (CONVANCE)] and FLAG antibody [ANTI-FLAG M2 Monoclonal Antibody (SIGMA)] ; and a secondary antibody used herein was anti-mouse IgG-HRP [Anti-Mouse IgG (H&L) HRP-Linked Antibody ( Cell Signaling TECHNOLOGY)]. To perform immuno-staining with 2 types of antibodies, HA antibody and FLAG antibody, two membranes to which the same protein solution had been blotted were prepared, and then immuno-staining was performed separately with the 2 types of antibodies. Thus, a specific band was detected only for a strain expressing the fusion protein when the cell wall fraction was treated with mild alkali. This result reveals that Pir1-HA-Kre2 fusion protein and Pir2-FLAG-Mnn1 fusion protein were localized simultaneously on a cell wall with a binding pattern (to a cell wall) representing the characteristics of PIR.

### Example 13 (Measurement of sequential transfer reaction of mannose)

Mannosyltransferase activity was measured by referring to Lussier et al's method (Lussier et al., JBC., 271, 11001-11008 (1996)). As an enzyme source, the yeast intact cell itself [ W303- YEp352GAP-II(PIR1-HA-KRE2), YEp351GAP-II(PIR2-FLAG-MNN1)] prepared in Example 12 was used. A control strain used herein was W303-YEp352GAP-II. As an acceptor substrate, PA-mannobiose was used; as a donor substrate, GDP-mannose was used. A reaction solution 50µl [100mM HEPES(pH7.2), 1mM MnCl₂, 5mM GDP-mannose, 300pmol PA-mannobiose] was prepared to contain 20µl of a cell suspension, followed by incubation at 37°C for 3 hours. The cell suspension used herein was prepared by collecting 1ml of a culture solution with OD600=4, washing twice with Wash Buffer [ 10mM Tris-HCl(pH8), 1mM PMSF], and then suspending in 20µl of Wash Buffer. Then, 50µl of ice-cooled water was added to the reaction solution. The precipitated cells were removed by centrifugation at 3,000rpm for 3 min, and then the supernatant with a molecular weight of 10,000 or more was removed with an Ultra Free (0.22µm). Then, mannobiose (disaccharide), mannotriose (trisaccharide) and mannotetraose (tetraose) were detected with HPLC. Amide-80 column (TSK gel An Amide-80, TOSOH, 0.46cm in diameter x 25cm in length) was used for HPLC. A mixture A containing 200mM acetic acid-triethylamine buffer (pH 7.0) and acetonitrile (10 : 90), and a mixture B containing 200mM acetic acid-triethylamine buffer (pH 7.0) and acetonitrile (60 : 40) were prepared. The column had been previously equilibrated by running the solvent A through the column at a flow rate of 1.0ml/min. Immediately after injection of samples, the proportion of the solvent B was raised linearly for 60 min to 100%, so that PA-oligosaccharide was eluted.

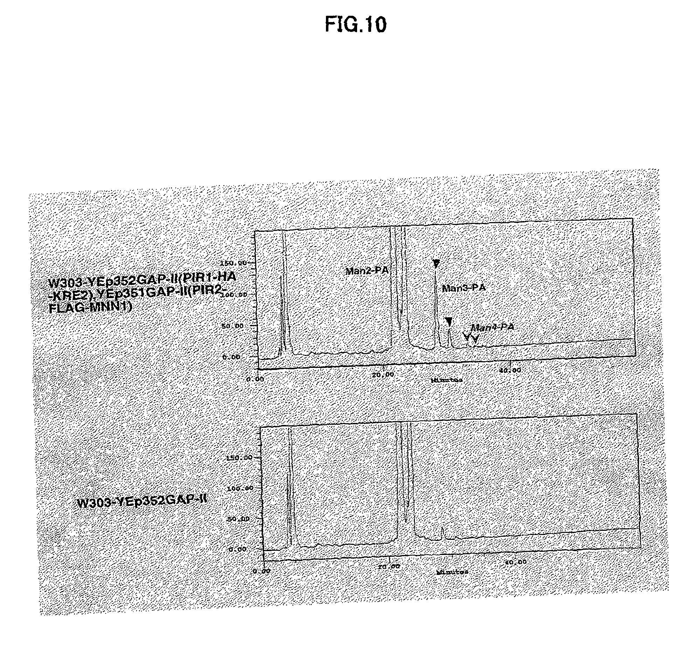

As a result, peaks indicating trisaccharide (mannotriose) and tetraose (mannotetraose) were detected only for the strain W303- YEp352GAP-II(PIR1-HA-KRE2), YEp351GAP-II (PIR2-FLAG-MNN1) that can simultaneously express both fusion proteins, Pir1-HA-Kre2 and Pir2-FLAG-Mnn1 (Fig. 10). Regarding the strain W303-YEP3S2GAP-II that expresses no fusion gene, almost no peak indicating trisaccharide (mannotriose) and tetraose (mannotetraose) was observed (Fig. 10).

These results suggest that integration of PIR1 and PIR2 as anchor proteins to a cell wall onto the N-terminal side of a useful protein enables presentation of the useful protein on the yeast cell surface layer. Moreover, the results also suggest that simultaneous expression of the above fusion proteins can easily cause a complex sequential reaction of enzyme on a yeast cell surface layer.

All publications, patents and patent applications cited herein are incorporated herein by reference in their entirety.

### Effect of the invention

The present invention enables immobilization of a useful protein, such as a glycosyltransferase, onto the surface of a yeast cell without deteriorating its enzyme activity, so that the invention can provide the immobilized protein as an immobilized enzyme. Since a process for purifying enzymes and a process for immobilizing enzymes to beads can be omitted, immobilized enzyme can be produced very easily and in large quantities.

## Claims

1. A fusion gene expression vector which contains a fusion gene comprising a gene encoding a useful protein downstream of a gene encoding the following yeast cell wall protein (a) or (b):
(a) a protein having an amino acid sequence represented by SEQ ID NO: 1; or
(b) a protein having an amino acid sequence derived from the amino acid sequence represented by SEQ NO: 1 by deletion, replacement, or addition of one or more amino acids, and having ability to be localized and immobilized on a yeast cell wall.

2. The fusion gene expression vector of claim 1 wherein the useful protein is a glycosyltransferase protein.

3. A transformant yeast which is transformed by the fusion gene expression vector of claim 1 or 2.

4. A method for producing an immobilized enzyme which comprises culturing the transformant yeast of claim 3, expressing a fusion gene on the surface layer of a yeast cell wall, and obtaining yeast that contains a useful protein immobilized on the cell wall.

5. An immobilized enzyme which is obtained by the method of claim 4.

6. The immobilized enzyme of claim 5 wherein the enzyme to be immobilized is a glycosyltransferase.

7. A method for producing a sugar chain or sugars which employs the immobilized enzyme of claim 6.

8. A fusion gene expression vector which contains a fusion gene comprising a gene encoding a useful protein downstream of a gene encoding the following yeast cell wall protein (a) or (b) as follows:
(a) a protein having an amino acid sequence represented by SEQ ID NO: 2;
(b) a protein having an amino acid sequence derived from the amino acid sequence represented by SEQ NO: 2 by deletion, replacement, or addition of one or more amino acids, and having ability to be localized and immobilized on a yeast cell wall.

9. The fusion gene expression vector of claim 8 wherein the useful protein is a glycosyltransferase protein.

10. A transformant yeast which is transformed with the fusion gene expression vector of claim 8 or 9.

11. A method for producing an immobilized enzyme which method comprises culturing the transformant yeast of claim 10, expressing a fusion gene on the surface layer of a yeast cell wall, and obtaining yeast that contains a useful protein immobilized on the cell wall.

12. An immobilized enzyme which is obtained by the method of claim 11.

13. The immobilized enzyme of claim 12 wherein the enzyme to be immobilized is a glycosyltransferase.

14. A method for producing a sugar chain or sugars which employs the immobilized enzyme of claim 13.

15. A transformant yeast which is transformed by allowing the yeast to contain at least two or more types of the fusion gene expression vectors of claims 1 and 8.

16. A method for producing an immobilized enzyme which comprises culturing the transformant yeast of claim 15, expressing simultaneously fusion genes on the surface layer of a yeast cell wall, and obtaining yeast that contains at least two or more types of useful proteins immobilized on the cell wall.

17. An immobilized enzyme which is obtained by the method of claim 16.

18. The immobilized enzyme of claim 17 wherein the enzymes to be immobilized are two or more types of glycosyltransferases.

19. A method for producing a sugar chain or sugars which sequentially converts sugar chains or sugars using the immobilized enzymes of claim 18.
